# EUROPEAN PATENT SPECIFICATION

(11) **EP 4 349 334 B1**
(45) Date of publication and mention of the grant of the patent: **19.03.2025**
(21) Application number: 22306512.9
(22) Date of filing: 07.10.2022
(51) Int. Cl.: A61K 31/445, A61K 9/00, A61K 9/08, A61K 47/12, A61P 3/00, A61K 47/22, A61K 47/26, A61K 47/10

(54) **PALATABLE LIQUID SOLUTION CONTAINING HIGH CONCENTRATION OF MIGLUSTAT**
WOHLSCHMECKENDE FLÜSSIGE LÖSUNG MIT HOHER MIGLUSTATKONZENTRATION
SOLUTION LIQUIDE AGRÉABLE CONTENANT UNE CONCENTRATION ÉLEVÉE DE MIGLUSTAT

(43) Date of publication of application: 10.04.2024
(73) Proprietor: Theranexus, 69008 Lyon (FR)
(72) Inventor: DUCHÊNE, Adeline, 02270 Crécy-sur-Serre (FR); VEYS, Julien, 92130 Issy-les-Moulineaux (FR); CHARVERIAT, Mathieu, 92140 Clamart (FR); ANAGONOU, Melanie, 75020 Paris (FR)
(74) Representative: Regimbeau

(56) References cited:
- EP-A1- 3 944 858
- US-A1- 2010 022 620

## Description

### SUMMARY

The present invention relates to a stable and palatable formulation containing high amount of miglustat, that can be used especially by children or people having difficulties to swallow. This highly concentrated formulation contains at least 150 mg/mL of Miglustat, and, as excipients, sodium benzoate, tartaric acid, a sweetener, and optionally an acidic flavour. This formulation can be advantageously administered along with therapeutic disaccharidase enzyme activators, so as to alleviate the intestinal side effects of miglustat.

### DESCRIPTION OF THE PRIOR ART

Miglustat (1,5 [butylimino]-1,5-dideoxy-D-glucitol) is a single stereoisomer synthetically derived from an imino sugar extracted from plants and microorganisms. It has a molecular weight of 219 and is a white crystalline substance that is highly soluble in water (>1 g/mL at ambient temperature). It is used to treat mild to moderate type 1 Gaucher disease (GD1) when enzyme replacement therapy is unsuitable, and Niemann-Pick disease type C (NP-C), which are both inherited metabolic disorders (Riahi et al, 2015). It has also recently been shown to be a promising candidate for the treatment of the Batten disease (or JNCL or CLN3 disease) (WO 2022/023573) or auto-immune diseases (US 2010/022620).

The standard adult Miglustat dose for GD1 is 100 mg three times a day. For NP-C, the standard dose for adults and adolescents is 200 mg three times a day. For younger patients under the age of 12, the dose should be adjusted on the basis of their body surface area.

Yet, Zavesca^{®} is currently only formulated as capsules of 100 mg. These capsules are not appropriate for children, because i) these are highly loaded with the active ingredient, such load being not appropriate for their body surface and ii) capsules cannot be easily swallowed by children or ill patients experiencing dysphagia. In this context, there is a need to reformulate Zavesca^{®} into a swallowable formulation, e.g., a liquid formulation which volume would depend on the patient's corpulence.

Yet, the decision to reformulate Zavesca^{®} for the purpose of administration via alternative routes (i.e., in liquid, orally, or via nasogastric tube) is currently at the discretion of the prescribing physician. The process of reformulating Zavesca^{®} at different dosage strengths can only be done by an experienced and qualified healthcare professional to ensure the safe administration of the required dose.

In this context, there is therefore a need in the art to provide a liquid formulation of Zavesca^{®} which can be easily available and can be swallowed by patients who have difficulty to swallow and / or that need particular dosage of the molecule (in particular children).

Although the need is great to dispose of such a liquid formulation, there are however a number of unsolved problems that are still associated with this kind of formulation.

A first problem that arises from reformulating Zavesca^{®} in a liquid solution is that Miglustat has a very bitter taste that is generally regarded as inedible. Because of this intolerable taste, Miglustat cannot be simply added in a fruit-puree or in a beverage, these becoming instantaneously inedible as soon as Miglustat is added. It is therefore necessary to associate the molecule with flavorings and/or sweeteners that will mask this prejudicial taste and render the absorption pleasant for the patient, in particular for children.

A second problem is linked to the well-known risk of gastrointestinal side effects that are usually associated with Zavesca^{®} uptake. As a matter of fact, when orally administered, Miglustat reaches a high concentration in the intestinal lumen, where it inhibits the disaccharidases and impairs the cleavage of carbohydrates which therefore abnormally accumulate. This accumulation of maldigested carbohydrates is associated with osmotic influx of water, increased fermentation activity of the commensal bacteria and production of irritating metabolites in the intestinal lumen, which lead to the appearance of gastrointestinal intolerances (Amiri M. et al, J. Inherit Metab Dis (2012). In view of this problem, it is not possible to use complex carbohydrates, in particular sugar, sorbitol or mannitol, as well as milk (unless lactose-free), in a treatment containing this molecule. Thus, there is a need to identify an excipient that efficiently mask the bitterness of high doses of Miglustat, rendering the liquid formulation pleasant to be swallowed by children, said excipient being not a complex carbohydrate.

A third problem is linked to the poor stability of Zavesca^{®} when formulated in a liquid suspension. As a matter of fact, it has been reported that a suspension of Zavesca^{®} with a final concentration of Miglustat 20mg/mL becomes brown even when stored in a refrigerated area, unless the pH of the suspensions is kept very acidic (Riahi et al, 2015). That's why it was recommended to reformulate Miglustat only extemporaneously, by dissolving Miglustat in water (see e.g. EP 3 944 858) or pure unsweetened fruit juice immediately prior to administration and that the solution should not be stored (Janssen scientific affairs document on the compounding of miglustat). This could possibly not be achieved by a health practitioner on a daily basis.

There is therefore a need to dispose of a liquid solution of Miglustat that is stable at room temperature for months, so that it can be easily transported and taken at any time during the day, even at school. Also, the liquid solution should ideally remain microbiologically safe even after the bottle is opened several times per day over a period that could extend beyond 2 months.

A fourth problem is due to the fact that the ideal Zavesca^{®} solution should be much more concentrated than the 20 mg/mL Miglustat reported in the prior art, so that the volume to be administered to the patients could be smaller. As a matter of fact, 0.8mL of a 250 mg/mL solution would be easier to swallow than 10 mL of a 20 mg/mL solution. As the liquid solution is dedicated to patients that have difficulty to swallow, the smaller the volume is, the better the compliance will be. Using a smaller volume will however not solve the bitterness problem, since the bitterness is the same in a 10 mg/mL solution and in a 250 mg/mL solution, and it is strongly perceivable even in a 0.8mL volume.

Altogether, the technical problem which is herein posed was therefore to provide a liquid solution of Miglustat, that would be suitable for patients that have difficulty to swallow and / or that need particular dosage of the molecule (in particular children), said liquid solution containing at least 150 mg/mL of Miglustat, but at the same time being i) not bitter, ii) microbiologically stable in time (for at least one month in a non refrigerated area) and iii) containing no carbohydrate (to avoid gastrointestinal side effects).

To the inventor's knowledge, very few liquid solutions containing Miglustat were proposed and studied in the art. In fact, only two proposals of liquid formulation containing Miglustat have been published so far:
On a one hand, Riahi et al, 2015 described the stability of Zavesca^{®} dissolved in InOrpha^{®} suspending vehicle, a liquid suspending excipient for compounding of oral solutions and suspensions. The authors reported that the contents of Zavesca^{®} 100 mg capsules were transferred into InOrpha^{®}. Although Zavesca^{®} was soluble in InOrpha^{®} at all concentrations tested, some of the excipients were not. An InOrpha^{®} suspension containing 20 mg/mL Zavesca^{®} was investigated initially. Subsequently, a pH-adjusted suspension of 20 mg/mL, and non- adjusted 10 and 5 mg/mL suspensions were evaluated. All suspensions were stored under refrigerated conditions. Physicochemical and microbiological challenge testing was performed at 0 hours and after 14 and 28 days. Degradation was assessed by high- performance liquid chromatography, appearance was assessed visually, and pH was recorded. Additionally, suspensions were inoculated with seven species of bacteria, yeast, and mold, and growth evaluated using membrane filtration. Zavesca^{®} 20 mg/mL suspension changed from yellow (0 hours) to brown (days 14 and 28); pH remained stable at 7.4-7.6. Pure InOrpha^{®} (pH 4.6) remained yellow throughout the study. Pure InOrpha^{®} adjusted to pH 7.5 displayed a brownish discoloration after 9 days Zavesca^{®} 5 and 20 mg/mL suspensions, adjusted to pH 6.5 and 4.4, respectively, remained yellow at days 14 and 28. Zavesca^{®} 10 mg/mL suspension (pH 7.3) changed from yellow to brown on day 9. Yet, the concentration of Miglustat in these stable solutions was too low to be used in small quantity, in particular to be swallowed by children and/or patients presenting with dysphagia. Moreover, the bitter taste of Miglustat was not tested nor masked. This liquid solution therefore does not solve the technical problem of the invention.

On the other hand, the AMICUS patent application WO2014/110270 disclosed parenteral formulations include 1-DNJ (in particular Miglustat), or a salt or derivative thereof, suspended in a buffer which is selected from sodium citrate, sodium acetate and sodium phosphate. In this formulation, the concentration of the Miglustat in the formulation is low (within the range from about 10 mg/ml to about 100 mg/ml, or from about 20 mg/mg to about 50 mg/ml, or from about 20 mg/ml to about 35 mg/ml). This formulation is to be used intravenously or subcutaneously. This liquid solution therefore does not solve the technical problem of the invention.

### DETAILED DESCRIPTION OF THE INVENTION

The formulation is composed of the active product ingredient miglustat, known for its competitive inhibition of the enzyme glucosylceramide synthase and also for the inhibition of disaccharidases which is suspected to induce gastrointestinal adverse effects.

In order to define the criteria of selection of the components of the product of the invention, different objectives were set:
- Dosage form and route of administration : the proposed drug product label is in particular targeting children from birth or patients presenting with dysphagia. Hence an oral liquid formulation is more appropriate for a precise dosage (per mg/m² of body surface area or mg/kg of body weight)
- Dosage strength : a high concentration of miglustat would allow to reduce the intake volume for children and to mix it easily with a fruit juice if needed
- Palatability : an oral paediatric solution requires to be easy to swallow and have an acceptable taste for children,
- Stability : as the drug product will be administered several times per day, the formulation must have an antimicrobial activity allowing a long preservation, even when the drug container is opened several times per day.

The profile analysis of miglustat at the target dose by the e-tongue system demonstrated that miglustat taste might be close to a salty and bitter taste. Considering the cited objectives, a focus must be made particularly on masking the well-known bitterness taste of miglustat to propose an acceptable drug product for pediatric patients.

Intended administration with multiple dosage of the oral solution require a maintenance of antimicrobial effect. To this purpose, two preservatives were tested in combination with miglustat (sodium benzoate and sodium metabisulphite). A change of colour after different storage conditions of the sodium metabisulphite formulation could be interpreted as a lack of stability or compatibility between the tested preservative and miglustat. Moreover, the evaluation of related impurities demonstrated an increase in the total amount of impurities after different storage conditions for sodium metabisulphite formulations which could imply risk of stability of the formulations. The formulation with a combination of sodium benzoate and miglustat remained clear, colourless and with an amount of related impurities after the described storage conditions relatively similar to initial measurements.

Further product development has hence been conducted with sodium benzoate that requires a specific pH of the solution between 3 and 5 to display its preservative effect in the formulation.

To ensure sodium benzoate efficacy, maintaining the solution between a pH of 3 and 5 is essential. Moreover according to miglustat profile with the e-tongue experiment, miglustat appears to be highly bitter; the acidity of the additive could counterbalance the bitterness of its taste.

To this aim, the compatibility of miglustat with several acids was tested. Whereas ascorbic acid and/or ascorbate are usually used for this purpose, an unexpected unknown impurity appeared for all three formulations at 60°C (1 week) and 40°C (2 weeks). Moreover, sodium ascorbate, ascorbic acid or both combined to miglustat induced a coloration of the solutions at all tested storage condition (60°C, 40°C/75%RH and 25°C/60%RH up to 4 weeks). Surprisingly, the combination of tartaric acid and miglustat demonstrated no degradation of the solutions at any storage conditions nor particular changes in related impurities amount.

According to compatibility results, tartaric acid was selected to maintain an acidic pH between 3 and 5 for preservative efficacy purpose. Moreover, the adjunction of an acidic buffer would help mask the bitterness of the solution.

According to miglustat profile from the e-tongue analysis, the addition of sugar could decrease the bitter taste and thereby improve the acceptance of miglustat. However, gastrointestinal adverse events, mainly diarrhoea, were observed in more than 80% of patients taking Zavesca^{®}. The suspected mechanism of action is the inhibition of intestinal disaccharidases by miglustat as sucrase isomaltase which reduces the absorption of disaccharides. Clinical practice demonstrated that GI events were responsive to a reduction in the consumption of saccharose, lactose and other carbone hydrates, otherwise a temporary reduction in the posology could be necessary (Zavesca SmPC).

Thus, no complex sugar can be used to enhance the sweetness of the formulation such as sucrose or polyols in order to avoid the increase of GI effects. The drug compatibility of aspartame and acesulfame potassium were thus tested with miglustat. At the tested storage conditions, solution with aspartame was clear but the one with acesulfame potassium was pale yellow. Moreover, aspartame+miglustat formulation presented a high increase in the amount of related impurities compared to the initial measure whereas acesulfame potassium+miglustat related impurities were complex to identify and no suitable analytical method could be developed. Hence, potassium acesulfame was selected for the next step. In case of failure, a mix of glycerin and monoammonium glycyrrhizate in a 10/1 ratio was added to the list of sweeteners to test.

Unexpectedly, during the product development studies, a precipitation of the formulations containing acesulfame potassium was observed. The adjustment of pH was suspected as the precipitation appeared when the concentration of acidic buffer was corrected to obtain a pH below 5. This precipitation was not observed with the use of a mix of glycerin and monoammonium glycyrrhizate in a 10/1 ratio without acesulfame potassium. The mix of glycerin and monoammonium glycyrrhizate in a 10/1 ratio was therefore selected as a sweetener.

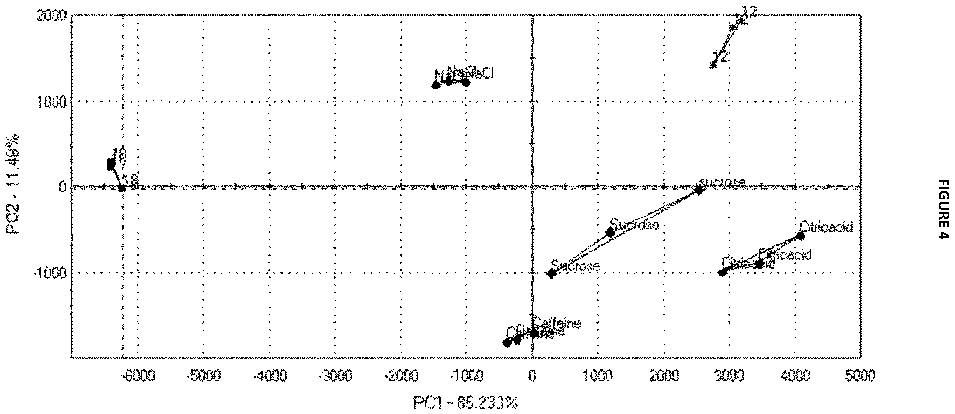

In the formulation according to the invention, the bittersweet taste of Miglustat was managed with increasing the proportion of sweet taste and acid despite the challenges cited above (see Figure 4).

In a first aspect, the present invention targets an acidic liquid formulation for oral administration, said formulation containing at least 150 mg/mL of Miglustat, said liquid formulation containing :
- sodium benzoate as preservative,
- tartaric acid so as to maintain the pH of the formulation comprised between 3 and 5,
- a sweetener, and
- optionally a flavour, advantageously an acidic flavour.

### Nature of the ingredients

The formulation of the invention contains at least 150 mg/mL of Miglustat, preferably at least 200 mg/mL of Miglustat, more preferably at least 250 mg/mL of Miglustat, even more preferably between 250 mg/mL and 300mg/mL (included).

The term "Miglustat" refers to the compound N-butyl-deoxynojirimycin (N-butyl DNJ), also named 1,5-(butylimino)-1,5-dideoxy-D-glucitol, N-butyl-deoxynojirimycin or (2R,3R,4R,5S)-1-butyl-2-(hydroxymethyl)piperidine-3,4,5-triol, as well as a salt thereof, provided said salt is capable of inhibiting the glucosylceramide synthase enzyme efficiently.

Sodium benzoate (CAS 532-32-1) is the sodium salt of benzoic acid. It is widely used as a food preservative (with an E number of E211) and a pickling agent. It appears as a white crystalline chemical with the formula C6H5COONa. It is also possible to use the benzoic acid (E210) in the composition of the invention.

Tartaric acid (CAS 87-69-4, 2,3-Dihydroxybutanedioic acid) is an alpha-hydroxy-carboxylic acid, is diprotic and aldaric in acid characteristics, and is a dihydroxyl derivative of succinic acid. It has two enantiomers: (2R,3R)-tartaric acid (L-(+)-tartaric acid), and (2S,3S)-tartaric acid (D-(-)-tartaric acid). Both of them can be used in the composition of the invention. The tartrate salt can be also used.

In a preferred embodiment said sweetener is a mix of glycerin and monoammonium glycyrrhizate in a 10/1 ratio, for example the sweetener known as "Magnasweet MM110". It is formulated as a liquid solution. This sweetener is preferably used in an amount of 0.01% to 10% by weight (weight / weight), preferably from 1% to 10% by weight, more preferably from 2% to 7% by weight.

The liquid formulation of the invention can contain a "flavour". A flavour, as used in the pharmaceutical industry, refers to either natural or artificial tastes, which may include fragrances and colours. In particular, flavours can be used for orally consumed products such as syrups, chewable tablets, suspensions, or gums in order to make the bitter taste of the medicine more palatable.

The formulation of the invention may contain any flavour that is palatable for the treated patients. For example, said flavour can be chosen in the group consisting of: ananas, anise, apple, apricot, banana, blackberry, blueberry, caramel, cherry, chocolate, cocoa, coconut, coffee, cola, cranberry, blackcurrant (cassis), redcurrant (or red currant), grape, grapefruit, grenadine, lemon, maple, mint, orange, walnut, passion fruit, peach, pear, pineapple, plum, prune, raspberry, strawberry, tangerine, tutti frutti, vanilla, etc.

In a preferred embodiment, the flavour used in the formulation of the invention is red currant or orange.

The formulation of the invention also contains a "pharmaceutically acceptable carrier" or "excipient" or "solution", which is a non-toxic liquid filler, diluent, or formulation auxiliary of any type. In a preferred embodiment, the formulation of the invention is an aqueous formulation and thus contains water as pharmaceutically acceptable excipient (typically between about 50% and 90% of water).

### Dosages

In a preferred embodiment, the liquid formulation of the invention contains between 0.5 and 100 mg/mL of sodium benzoate.

In a preferred embodiment, the liquid formulation of the invention contains between 50 and 200 mg/mL of tartaric acid.

In a preferred embodiment, the liquid formulation of the invention contains between 0.1% to 10% by weight of a sweetener, preferably of a liquid mix of glycerin and monoammonium glycyrrhizate in a 10/1 ratio. Said liquid mix is for example Magnasweet MM110.

In a preferred embodiment, the liquid formulation of the invention contains between 0.1 and 10 % of an acidic flavour.

Altogether, the formulation of the invention preferably contains
- at least 150 mg/mL of Miglustat,
- between 0.5 and 100 mg/mL of sodium benzoate,
- between 50 and 200 mg/mL of tartaric acid, so that the final pH of the formulation is comprised between 3 and 5,
- between 0.1% to 10% of a mix of glycerin and monoammonium glycyrrhizate in a 10/1 ratio, and
- between 0.1 and 10 % of red currant or orange flavour.

It is noteworthy that the formulation of the invention should not contain any carbohydrates, because the active ingredient miglustat is known for its competitive inhibition of gut disaccharidases, what triggers gastrointestinal adverse effects.

Also, it is not recommended to add any taste enhancer in the formulation, since it may increase the bitterness of miglustat and therefore render the formulation more bitter and less palatable.

### Viscosity

The formulation of the invention is preferably a liquid solution having a low viscosity comprised between 1 and 20 mPa.s⁻¹, when measured in "standard conditions" (20°C, 1 atm.).

It can also be a little viscous and therefore have a higher viscosity comprised between 20 and 60 mPa.s⁻¹, when measured by a viscometer in "standard conditions" (20°C, 1 atm.).

### Stability

The formulation of the invention should be sufficiently stable to be used for several weeks, preferably at room temperature, after the container is opened.

In a preferred embodiment, the formulation of the invention remains unaltered (in taste, colour and microbiological composition) during several months at room temperature when the product is stored before being sold, and for at least two weeks, preferably three weeks, more preferably one month, at room temperature.

The inventors demonstrate in the examples below that the formulation of the invention is indeed able to stay unaltered in conditions of high humidity (up to 75% RH, and high temperature (up to 40°C) for at least 4 weeks (tables 9-16).

### Therapeutic uses

In a second aspect, the present invention relates to the liquid formulation as described above, for use as a medicament.

This medicament or liquid formulation of the invention is for use in the treatment of lysosomal storage disorders.

Non-limiting examples of lysosomal storage disorders and disorders characterized by lysosomal dysfunction that may be treated using the liquid formulation of the invention include : Juvenile Neuronal Ceroid Lipofuscinosis (JNCL, juvenile Batten or CLN3 disease), Aspartylglucosaminuria, Cystinosis, Fabry Disease, San Filippo disease, Gaucher Disease Types I, II, and III, Glycogen Storage Disease II (Pompe Disease), GM2-Gangliosidosis Type I (Tay Sachs Disease), GM2-Gangliosidosis Type II (Sandhoff Disease), Metachromatic Leukodystrophy, Mucolipidosis Types I, II/III and IV, Mucopolysaccharide Storage Diseases, Niemann-Pick Disease Types A/B, C1 and C2, Schindler Disease Types I and II, CLN1 disease, CLN2 disease, CLN4 disease, CLN5 disease, CLN6 disease, CLN7 disease, CLN8 disease, CLN10 disease, CLN11 disease, CLN12 disease, CLN13 disease, and CLN14 disease.

The mucopolysaccharide storage disease is preferably selected from : Hurler syndrome (MPS IH), Hurler-Scheie syndrome (MPS IH/S), Scheie syndrome (MPS IS; Mucopolysaccharidosis type V), Hunter syndrome (MPS II), Sanfilippo syndrome A (MPS IIIA), Sanfilippo syndrome C (MPS IIIC), Sanfilippo syndrome D (MPS IIID), Morquio Type A, Morquio Type B, Maroteaux-Lamy (MPS VI), Sly diseases (MPS VII), and Natowicz syndrome (MPS IX).

In a preferred embodiment, the formulation of the invention is for use in the treatment of Juvenile Neuronal Ceroid Lipofuscinosis (JNCL, juvenile Batten or CLN3 disease), Aspartylglucosaminuria, Cystinosis, San Filippo disease, Glycogen Storage Disease II (Pompe Disease), Metachromatic Leukodystrophy, Mucolipidosis Types I, II/III and IV, Mucopolysaccharide Storage Diseases, Niemann-Pick Disease Types A/B, C1 and C2, Schindler Disease Types I and II, CLN1 disease, CLN2 disease, CLN4 disease, CLN5 disease, CLN6 disease, CLN7 disease, CLN8 disease, CLN10 disease, CLN12 disease, CLN13 disease, and CLN14 disease.

In a preferred embodiment, the formulation of the invention is for use in the treatment of Neuronal Ceroid Lipofuscinosis (CLN disease), in particular CLN1 disease, CLN2 disease, CLN3 disease, CLN4 disease, CLN5 disease, CLN6 disease, CLN7 disease, CLN8 disease, CLN10 disease, CLN11 disease, CLN12 disease, CLN13 disease, or CLN14 disease.

In a most preferred embodiment, the formulation of the invention is for use in the treatment of Juvenile Neuronal Ceroid Lipofuscinosis (JNCL, or Batten disease, or the CLN3 form of Batten disease). Although it shares some pattern with other CLN diseases, this disease is still very complicated to understand, due to the fact that the CLN3 primary function is still unknown, and because this protein has multiple interaction partners (Getty A.L. and Pearce D.A., 2011).

JNCL is the most prevalent neurodegenerative disorder of childhood. A hallmark of JNCL is the intralysosomal accumulation of ceroid lipopigments in most nerve cells and in various extra-cerebral tissues, indicating impairment of autophagy-lysosome pathways. JNCL presents with vision failure and hearing loss, and progresses to include seizures, motor dysfunction, and dementia. JNCL patients experience relentless physical and cognitive decline that leads to death by the third decade of life. As such, treating JNCL using the formulation of the invention of the present invention will prevent intralysosomal accumulation of ceroid lipopigments in nerve cells and in various cerebral and extra-cerebral tissues of a subject having JNCL, or will reduce or eliminate intralysosomal accumulation of the ceroid lipopigments. Additionally, treating JNCL using the formulation of the invention will prevent, reverse, or arrest cognitive decline in a subject. Methods of determining cognitive decline resulting from JNCL in a subject are known in the art. For instance, treating JNCL using the formulation of the invention may prevent, reverse, or arrest vision failure. Treating JNCL using the formulation of the invention may also prevent, reverse, or arrest hearing loss. Treating JNCL using the formulation of the invention may also reduce the severity and/or intensity of seizures. Additionally, treating JNCL using the formulation of the invention may improve or prevent motor dysfunction. Treating JNCL using the formulation of the invention may also improve or prevent dementia.

In another embodiment, the formulation of the invention is for use to treat patients suffering from neurodegenerative disorders known to be associated with a lysosomal disorder, especially when they experience dysphagia.

In a preferred embodiment, the formulation of the invention is administered to human patients that have difficulties to swallow tablets or pills. It is particularly suited for patients presenting dysphagia, or conversely for children weighting less than 30kg.

In a preferred embodiment, the liquid formulation of the invention is administered to human patients suffering from a lysosomal storage disorder, preferably to patient with dysphagia or patient suffering from the Batten Disease.

As used herein, the term "treat" may be used to describe prophylaxis, amelioration, prevention or cure of a lysosomal storage disorder and disorders characterized by lysosomal dysfunction and/or one or more of its associated symptoms. For instance, treatment of an existing lysosomal storage disorder and disorders characterized by lysosomal dysfunction may reduce, ameliorate or altogether eliminate the disorder, or prevent it from worsening. Prophylactic treatment may reduce the risk of developing a disorder and/or lessen its severity if the disorder later develops.

### Kit of part with therapeutic disaccharidase enzymes

Treatment with Miglustat is often associated with occurrence of gastrointestinal side effects similar to carbohydrate maldigestion symptoms, like diarrhea, bloating, vomiting, and abdominal cramps. These side effects have been shown to be due to Miglustat's interference with carbohydrate digestion in the intestinal lumen, notably via reversible inhibition of disaccharidases that cleave α-glycosidically linked carbohydrates (Amiri M. et al, J. Inherit Metab Dis (2012)). Also, it has been shown more recently that Miglustat interferes with the N-glycosylation of sucrase-isomaltase in the endoplasmic reticulum, thereby delaying its intracellular trafficking and apical targeting (Amiri M. et al, J. Inherit Metab Dis (2014)).

Intestinal disaccharidases are integral membrane glycoproteins that are located in the brush border membrane of intestinal epithelial cells. Prominent members of this family of hydrolytic enzymes are: sucrase-isomaltase (SI), lactase- phlorizin hydrolase (LPH) and maltase-glucoamylase (MGA). These enzymes digest carbohydrates in the intestinal lumen and reveal preferential affinities toward cleavage of α- or β-glycosidic linkages. For example, the sucrase and isomaltase activities of SI as well as maltase and glucoamylase activities of MGA can hydrolase the α-glycosidic linkages of the major dietary carbohydrates such as starch, glycogen, sucrose and maltose. The resulting monosaccharides are eventually transported across the brush border membrane of epithelial cells into the cell interior.

When orally administered, Miglustat reaches a high concentration in the intestinal lumen, where it inhibits the disaccharidases and impairs the cleavage of carbohydrates which therefore abnormally accumulate. This accumulation of maldigested carbohydrates is associated with osmotic influx of water, increased fermentation activity of the commensal bacteria and production of irritating metabolites in the intestinal lumen, which lead to the appearance of gastrointestinal intolerances (Amiri M. et al, J. Inherit Metab Dis (2012).

The daily diet is normally composed of appreciable amounts of starch, sweets and soft drinks which are enriched in α-glycosidically linked sugars. Patients treated with Miglustat are therefore usually recommended to avoid absorbing carbohydrate containing meals during the few hours following Miglustat's intake, to avoid coincidence of these carbohydrate with Miglustat in the intestinal lumen (Belmatoug N et al, J. Inherit Metab Dis 2011).

Alternative treatments have been proposed to counteract the negative intestinal effect of an impairment of intestinal hydrolytic enzymes, in patients suffering from congenital sucrase-isomaltase deficiency (CSID) or in patients treated with Miglustat:
- Administering compounds that slow the intestinal transit, such as Loperamide or trimebutine (Lyseng-Williamson K.A. et al, Drugs 2014; Belmatoug N et al, J. Inherit Metab Dis 2011).
- Administering substitute disaccharidase enzymes by "Enzyme Replacement Therapy" (ERT). These substitute enzymes can be for example invertase (EC 3.2.1.26), sucrase-isomaltase (SI, EC 3.2.1.48 and EC 3.2.1.10 respectively), β-galactosidase (EC 3.2.1.21), lactase- phlorizin hydrolase (LPH, EC 3.2.1.108), sacrosidase (such as Sucraid^{®}), amylase (EC 3.2.1.1) and/or amyloglucosidase (EC 3.2.1.3), that help digesting saccharose and/or starch.
- Administering bacterial probiotics that express high amount of at least one of these disaccharidase enzymes, or that express enzymes stimulating the endogenous disaccharidase enzymes (Belmatoug N et al, J. Inherit Metab Dis 2011, Remenova et al, Journal of rare diseases, 2015).
- Administering yeast cells, for example lyophilized *Saccharmoyces cerevisiae* (LSC) or *Saccharomyces boulardii* cells, that express high amount of at least one of these disaccharidase enzymes, or that express enzymes stimulating the endogenous disaccharidase enzymes.

It is for example possible to counteract the intestinal side effects of Miglustat by administering yeast sucrase (Treem WR et al, 1993) and in particular baker yeast sucrase (sucrase enzyme from the *Saccharomyces Cerevisiae* yeast).

It is also possible to administer purified enzymes from the *Saccharomyces Cerevisiae* yeast, for example the invertase enzyme (EC 3.2.1.26) commercialized by Sigma-Aldrich (I4504).

All these enzyme replacement therapies have been already proposed for treating the patients suffering from congenital sucrase-isomaltase deficiency (CSID), who are known to experience the same intestinal disorders as Miglustat-treated patients. These treatments are therefore thought to alleviate the intestinal symptoms of patients treated with high doses of Miglustat (Belmatoug N et al, J. Inherit Metab Dis 2011, Remenova et al, Journal of rare diseases, 2015).

In this context, the present invention relates to a combination product comprising the formulation of the invention, on one hand, and an activator of intestinal disaccharidase enzymes, on another hand, for simultaneous, separated, or staggered use in the treatment of the disorders mentioned above, according to claim 15.

Non-limiting examples of lysosomal storage disorders and disorders characterized by lysosomal dysfunction that may be treated using this combination product include : Juvenile Neuronal Ceroid Lipofuscinosis (JNCL, juvenile Batten or CLN3 disease), Aspartylglucosaminuria, Cystinosis, Fabry Disease, San Filippo disease, Gaucher Disease Types I, II, and III, Glycogen Storage Disease II (Pompe Disease), GM2-Gangliosidosis Type I (Tay Sachs Disease), GM2-Gangliosidosis Type II (Sandhoff Disease), Metachromatic Leukodystrophy, Mucolipidosis Types I, II/III and IV, Mucopolysaccharide Storage Diseases, Niemann-Pick Disease Types A/B, C1 and C2, Schindler Disease Types I and II, CLN1 disease, CLN2 disease, CLN4 disease, CLN5 disease, CLN6 disease, CLN7 disease, CLN8 disease, CLN10 disease, CLN11 disease, CLN12 disease, CLN13 disease, and CLN14 disease.

The mucopolysaccharide storage disease is preferably selected from : Hurler syndrome (MPS IH), Hurler-Scheie syndrome (MPS IH/S), Scheie syndrome (MPS IS; Mucopolysaccharidosis type V), Hunter syndrome (MPS II), Sanfilippo syndrome A (MPS IIIA), Sanfilippo syndrome C (MPS IIIC), Sanfilippo syndrome D (MPS IIID), Morquio Type A, Morquio Type B, Maroteaux-Lamy (MPS VI), Sly diseases (MPS VII), and Natowicz syndrome (MPS IX).

In a preferred embodiment, the combination product of the invention is for use to treat Juvenile Neuronal Ceroid Lipofuscinosis (JNCL, juvenile Batten or CLN3 disease), Aspartylglucosaminuria, Cystinosis, San Filippo disease, Glycogen Storage Disease II (Pompe Disease), Metachromatic Leukodystrophy, Mucolipidosis Types I, II/III and IV, Mucopolysaccharide Storage Diseases, Niemann-Pick Disease Types A/B, C1 and C2, Schindler Disease Types I and II, CLN1 disease, CLN2 disease, CLN4 disease, CLN5 disease, CLN6 disease, CLN7 disease, CLN8 disease, CLN10 disease, CLN12 disease, CLN13 disease, and CLN14 disease.In a preferred embodiment, the combination product of the invention enables to treat Neuronal Ceroid Lipofuscinosis (CLN disease), in particular CLN1 disease, CLN2 disease, CLN3 disease, CLN4 disease, CLN5 disease, CLN6 disease, CLN7 disease, CLN8 disease, CLN10 disease, CLN11 disease, CLN12 disease, CLN13 disease, or CLN14 disease. In a most preferred embodiment, the combination product of the invention enables to treat Juvenile Neuronal Ceroid Lipofuscinosis (JNCL, or Batten disease, or the CLN3 form of Batten disease).

The term "activators of intestinal disaccharidase enzymes" herein designate any molecule (e.g., small chemical compounds or enzyme proteins) or organisms (e.g., bacterial or yeast cells) that can enhance the activity and/or the expression level of at least one intestinal disaccharidase enzyme chosen among: invertase (EC 3.2.1.26), maltase (EC 3.2.1.20), trehalase (EC 3.2.1.18), sucrase-isomaltase (SI, EC 3.2.1.48 and EC 3.2.1.10 respectively), β-galactosidase (EC 3.2.1.21), lactase-phlorizin hydrolase (LPH, EC 3.2.1.108), sacrosidase (such as Sucraid^{®}), amylase (EC 3.2.1.1) and amyloglucosidase (EC 3.2.1.3).

In one preferred embodiment, these activators are the enzymes themselves obtained by purification or recombinant synthesis. In one embodiment, only one type of enzyme is administered. In another embodiment, a combination of different types of enzymes (e.g., two or three substitute enzymes) is administered. All these enzymes should still be active once reaching the intestine of the patients.

In another preferred embodiment, these activators are bacterial probiotics or yeast cells that are known to express high amount of at least one of these intestinal hydrolytic enzymes.

In a more preferred embodiment, these activators are provided by administering the sucraid^{®} mixture or the Baker yeast *Saccharomyces Cerevisiae,* or the *Saccharomyces boulardii* yeast, through oral administration.

In a particular embodiment, in the combination product of the invention, the activator of the intestinal enzyme is administered few hours (typically one to three hours) before the formulation of the invention.

### FIGURE LEGENDS

Figure 1 A: Distances from each tested formulation to miglustat 250 mg/mL (Principal Component Analysis, PCA). B: PCA aromatic profile of miglustat 10mg/mL, miglustat 250mg/mL and formulation 12.
Figure 2: Aromatic profile of miglustat alone (formulation F-18), as compared to Sodium chloride, Caffeine, Sucrose and Citric acid.
Figure 3: Aromatic profile of formulation F-12 (formulation according to the invention), as compared to Sodium chloride, Caffeine, Sucrose and Citric acid.
Figure 4: PCA formulation 12 and 18 (miglustat at 250 mg/mL) compared to NaCl, caffeine, sucrose and citric acid at 10mg/mL. Distances between sucrose and citric acid are closer to formulation 12 compared to formulation 18.

### EXAMPLES

In order to develop a formulation indicated for a paediatric population, an oral formulation was more appropriate for a precise dosage regarding the weight and size of each patient and also to better control the therapeutic window. Hence, the selection of a dose would be easier within the margin between the therapeutic efficacy and the gastro-intestinal effects. However, an oral paediatric solution requires a certain palatability, namely, a solution easy to swallow and with an acceptable taste for the patient. This last point was very challenging as the variety of sugars usually used to sweeten a solution were to be avoided as patients taking miglustat suffer from gastro intestinal effects due to the inhibition of disaccharidases.

The examples below show how it was possible to identify the formulation of the invention, which is pleasant to swallow and stable in time, even if containing a high concentration of Miglustat. Furthermore, it can be administered to Batten patients without risking to increase the gastro-intestinal side effects of miglustat.

### Material & Methods

### Preparation 1: Drug excipient compatibility

This study was conducted to assess the compatibility of miglustat with excipient candidates that can be used in the liquid Miglustat product. Each different excipient was manually stirred with miglustat and stored into amber glass vials.

In order to achieve desired aspects of Miglustat oral solution, the drug-excipient study was conducted in different excipients (inactive ingredients) including viscosifier, preservatives, flavoring agents, sweetener, coloring agents, etc.

### Standard preparation:

5mg of Miglustat standard were accurately weighed and transferred into a clean and dry 50 mL volumetric flask. 30 mL of the diluent were added and sonicated to dissolve. Later, the diluent was further added to make up to the mark and mixed well.

### Sample preparation procedure:

The contents of the sample vials were carefully transferred to a clean and dried 250 mL volumetric flask using a funnel. The vials and caps were rinsed carefully with 5 mL diluent for 4-5 times and all the washings were transferred into the volumetric flask. About 150 mL of diluent was added, then vortexed for about 3 min, then diluted to volume with diluent and mixed well.

**Table 1 : Composition of tested solutions**

| **Composition** | **mg/ml** | **g/75 ml** |
|---|---|---|
| Miglustat | 300 | 22.5 |
| Miglustat + Sodium Ascorbate | 300 + 3.28 | 22.5 + 0.246 |
| Miglustat + Ascorbic acid | 300 + 6.1 | 22.5 + 0.457 |
| Miglustat + Sodium Ascorbate + Ascorbic acid | 300 + 3.28+ 6.1 | 22.5 + 0.246 + 0.457 |
| Miglustat + Tartaric Acid | 300 + 100 | 22.5 + 7.5 |
| Miglustat + Sodium Metabisulphite | 300 + 2 | 22.5 + 1.5 |
| Miglustat + Aspartame | 300 + 0.126 | 22.5 + 0.00945 |
| Miglustat + Acesulfame Potassium | 300 + 192 | 22.5 + 14.4 |
| Miglustat + Sodium Benzoate | 300 + 3 | 22.5 + 0.225 |
| Miglustat + Red currant | 300 + 7.5 | 22.5 + 0.562 |
| Miglustat + Black currant | 300 + 7.5 | 22.5 + 0.562 |

### Storage conditions:

Vials were stored in climatic chambers with different environmental conditions: 25°C/60% RH, 40°C/75% RH and 60°C for up to 4 weeks, in closed containers. (*RH = relative humidity*)

**Table 2: Storage conditions**

| | 1 week | 2 weeks | 3 weeks | 4 weeks |
|---|---|---|---|---|
| 25°C/60% RH | D | D, RS | D | D, RS |
| 40°C/75% RH | D | D, RS | D | D, RS |
| 60°C | D, RS | | | |

| | | | | |
|---|---|---|---|---|
| *D : physical description* *RS : investigation for related substances* | | | | |

At each timepoint, related substances analysis and physical descriptions were performed on stored samples.

### Physical description methodology

After the defined storage time, samples were exited from the climatic chambers. Operators observed the solution and described the appearance of the solution by visual inspection.

### Analytical methodology for related substances analysis and Physical description

Apothecon Pharmaceuticals Miglustat API Related substances method (QC-QCM-0778-01) was used for the analysis of the drug excipient compatibility samples, with the standard and sample preparation procedure. All the chromatographic conditions, mobile phase preparation, diluent and blank preparation were taken from Related substances method of QC-QCM-0778-01.

### Preparation 2: Taste evaluation by experimenters

### Preparation of miglustat solutions

A little of purified water (Cooper) was poured into a 10-ml volumetric flask. Under the safety weighing cabinet (SAFETECH, APTYS 550), 3 g of miglustat (Amino Chemicals) were weighed and added to the volumetric flask. Potassium acesulfame (Calenese) then aspartame (HSWT) were weighed and added (according to the formula). Afterwards, tastesense (Kerry) was weighed and added to the flask. According to the formula, corresponding flavour (raspberry, blackcurrant, redcurrant, grapefruit, cherry) was weighed and added (Firmenich or Givaudan). Finally, citric acid was added (according to the formula) and purified water was added at *quantum satis.* Each formula was placed under magnetic stirring for about 1 hour.

**Table 3: Formulation composition for taste evaluation**

| **Formulation code** | **632** | **126** | **028** |
|---|---|---|---|
| Miglustat | 3,000 g | 3,000 g | 3,000 g |
| Acesulfame potassium | 0,240 g | 0,240 g | 0,240 g |
| Aspartame | 0,080 g | 0,080 g | 0,080 g |
| Flavour | Red currant | Blackcurrant | Grapefruit |
| Flavour quantity | 20 µL | 13 µL | 0,010 g |
| Tastesense SR | 0,010 g | 0,010 g | 0,010 g |
| Purified water | Qsp 10 ml | Qsp 10 ml | Qsp 10 ml |

### Testing method

Thirty minutes before the session, experimenters were not allowed to drink anything but water. All formulations were blinded with codes and randomized for each experimenter following a Latin square design. Experimenters were asked to taste each formulation and, to give a global grade (0 to 20). After the test, experimenters properly rinse their mouth and respected a minimum of 10 minutes between each formulation. Means of all rates were calculated by a third party.

### Preparation 3: Turbidity evaluation and electronic tongue experimentation

### Preparation of miglustat solutions

Formulations were prepared with batch size ranging from 30 to 100 mL, according to the following compositions:

**Table 4: Composition of Miglustat solution**

| | *F-18* | *F-12* | *F-13* | *F-20* | *F-21* | *F-26* | *F-27* | *F-28* | *F-37* |
|---|---|---|---|---|---|---|---|---|---|
| | *mg*/*ml* | | | | | | | | |
| *Miglustat* | *250.00* | *250.00* | *250.00* | *250.00* | *250.00* | *250.00* | *250.00* | *250.00* | *250.00* |
| *Tartaric acid* | - | *150.00* | *150.00* | *100.00* | *100.00* | *88.20* | *88.20* | *88.20* | *88.20* |
| *Sodium benzoate* | - | *3.00* | *3.00* | *2.30* | *2.30* | *2.30* | *2.30* | *2.30* | *2.30* |
| *Acesulfame K* | - | - | *5.00* | - | - | *4.00* | *4.00* | *4.00* | - |
| *Taste sense* SR *plus LQD* | - | - | - | - | *1.00* | - | *1.00* | *1.00* | - |
| *Taste sense* SR *PDR* | - | - | - | *1.00* | - | *1.00* | - | - | - |
| *Magna sweet MM110* | - | *28.00* | - | *29.50* | *29.50* | - | - | *29.50* | - |
| *Red currant* | - | *6.0* | *6.0* | *13.0* | *13.0* | *13.0* | *13.0* | *13.0* | - |
| *Water* | *q.s* | *q.s* | *q.s* | *q.s* | *q.s* | *q.s* | *q.s* | *q.s* | *q.s* |

**Table 5: Composition of solution of reference - Caffeine**

| **Ingredients** | **mg/mL** |
|---|---|
| Caffeine | 10 |
| Water | q.s |

**Table 6: Composition of solution of reference - citric acid**

| **Ingredients** | **mg/mL** |
|---|---|
| Citric acid | 10 |
| Water | q.s |

**Table 7: Composition of solution of reference - sodium chloride**

| **Ingredients** | **mg/mL** |
|---|---|
| Sodium chloride | 10 |
| Water | q.s |

**Table 8: Composition of solution reference - sucrose**

| **Ingredients** | **mg/mL** |
|---|---|
| Sucrose | 10 |
| Water | q.s |

### Appearance analyze procedure

The test of the appearance of the solution was a visual observation. After the final mixing, the operator commented the clarity and aspect of the solution after visual inspection.

### Electronic tongue procedure

To investigate the taste of each trial formulation, an electronic tongue system was used (Astree, Alpha MOS). Samples of 30 mL were collected from solutions to be tested and transferred into the testing beakers. The set of sensor electrodes were dipped into each testing solution for 3 minutes for analysis. The sensors were washed with water between each measurement. For each tested solution, the sensors recorded a specific response (Alpha software, V12.0).

### Analysis method for formulation comparisons

To compare miglustat formulation trial batches to miglustat alone, miglustat formulations were prepared as described above. A triplicate of each solution was analyzed by the electronic tongue. The distance between the mean sensor response for each solution and the mean response for miglustat alone at 250 mg/kg were calculated by PCA using Alpha soft (Version 12.0).

### Profiling method analysis

Solutions of reference and Miglustat formulations were prepared as described above. Samples of 30 mL were analyzed by the electronic tongue system. A triplicate of each solution was analyzed by the electronic tongue. The distance between the mean sensor response for each reference and either miglustat alone or the formulation to be profiled were calculated (Alpha software, V12.0).

### Results

### Drug excipient compatibility results

### Physical description

Physical criteria (dissolution status, colour) of solution with miglustat 300mg/ml alone or combined with acidic buffer were evaluated. According to the results summarized in Table 9, all tested formulations were solutions after 4 weeks in a closest recipient at 40°C/75RH or 25°C/60%RH. Only formulation F with tartaric acid and formulation P with hydrochloric acid presented no variation in color.

**Table 9: Physical description for combination of active and inactive ingredients (acidic buffers)**

| **Formulations** | **Condition/ Physical Description Initial, 60°C, 40°C/75%RH and 25°C/60%RH (closed) of Active + Inactive Ingredient** | | | | | | | | | |
|---|---|---|---|---|---|---|---|---|---|---|
| | **Initial** | **60°C** | **40°C/75%RH** | | | | **25°C/60%RH** | | | |
| | | **1 week** | ***1 week** | **2 weeks** | ***3 weeks** | **4 weeks** | ***1 week** | **2 weeks** | ***3 weeks** | **4 weeks** |
| A-Miglustat | Clear Solution | Clear Solution | Solution | Clear solution | Solution | Clear solution | Solution | Clear solution | Solution | Clear solution |
| B-Miglustat + Sodium Ascorbate | Clear Solution | Pale brown color Solution | Solution | Pale brown color solution | Solution | Pale brown color solution | Solution | Pale brown colour solution | Solution | Pale brown colour solution |
| C-Miglustat + Ascorbic acid | Clear Solution | Pale brown color Solution | Solution | Pale brown color solution | Solution | Pale brown color solution | Solution | Pale brown colour solution | Solution | Pale brown colour solution |
| D-Miglustat + Sodium Ascorbate + Ascorbic acid | Clear Solution | Pale brown color Solution | Solution | Pale brown color solution | Solution | Pale brown color solution | Solution | Pale brown colour solution | Solution | Pale brown colour solution |
| F-Miglustat + Tartaric Acid | Clear Solution | Clear Solution | Solution | Clear Solution | Solution | Clear Solution | Solution | Clear solution | Solution | Clear solution |
| P-Miglustat + Hydrochloric acid | Clear Solution | Clear Solution | Solution | Clear Solution | Solution | Clear Solution | Solution | Clear solution | Solution | Clear solution |

| | | | | | | | | | | |
|---|---|---|---|---|---|---|---|---|---|---|
| ** All the Samples marked with*(1^{st} week and 3^{rd} week samples) were observed for physical description Superficially as samples are loaded in amber colored bottles. All the samples were found to be like solution only, but in few Samples precipitation and gel formation was observed. But color of the solution was not visible as they were amber colored.* | | | | | | | | | | |

Physical criteria (dissolution status, colour) of solution with miglustat 300mg/ml alone or combined with preservatives were evaluated. According to the results summarized in Table 10, formulation I with sodium benzoate remained a clear solution after 4 weeks in a closed recipient at 40°C/75RH or 25°C/60%RH and also remained clear after 1 week at 60°C. Formulations H with sodium metabisulphite presented a pale-yellow color at every storage conditions even after 1 week at 60°C.

Unexpectedly, it was observed that the ascorbic acid was not compatible with Miglustat, as the solution containing both did not remain clear, as shown in table 13. An oxidation was suspected.

**Table 10: Physical description for combination of active and inactive ingredients (preservatives)**

| **Formulations** | **Condition/ Physical Description Initial, 60°C, 40°C/75%RH and 25°C/60%RH (closed) of Active + Inactive Ingredient** | | | | | | | | | |
|---|---|---|---|---|---|---|---|---|---|---|
| | **Initial** | **60°C** | **40°C/75%RH** | | | | **25°C/60%RH** | | | |
| | | **1 week** | ***1 week** | **2 weeks** | ***3 weeks** | **4 weeks** | ***1 week** | **2 weeks** | ***3 weeks** | **4 weeks** |
| I-Miglustat + Sodium Benzoate | Clear Solution | Clear Solution | Solution | Clear Solution | Solution | Clear Solution | Solution | Clear solution | Solution | Clear solution |
| H-Miglustat + Sodium Metabisulphite | Clear Solution | Pale yellow color solution | Solution | Pale yellow color solution | Solution | Pale yellow color solution | Solution | Pale yellow colour solution | Solution | Pale yellow colour solution |

Physical criteria (dissolution status, colour) of solution with miglustat 300mg/ml alone or combined with sweeteners were evaluated. According to the results summarized in Table 11, formulation K with aspartame remained a clear solution at all storage conditions. Formulations L with acesulfame potassium presented a pale yellow color at 40°C/75%RH after 2 and 4 weeks of storage.

**Table 11: Physical description for combination of active and inactive ingredients (sweeteners)**

| **Formulations** | **Condition/ Physical Description Initial, 60°C, 40°C/75%RH and 25°C/60%RH (closed) of Active + Inactive Ingredient** | | | | | | | | | |
|---|---|---|---|---|---|---|---|---|---|---|
| | **Initial** | **60°C** | **40°C/75%RH** | | | | **25°C/60%RH** | | | |
| | | **1 week** | ***1 week** | **2 weeks** | ***3 weeks** | **4 weeks** | ***1 week** | **2 weeks** | ***3 weeks** | **4 weeks** |
| K-Miglustat + Aspartame | Clear Solution | Clear Solution | Solution | Clear Solution | Solution | Clear Solution | Solution | Clear solution | Solution | Clear solution |
| L-Miglustat + Acesulfame Potassium | Clear Solution | Clear Solution | Solution | Pale yellow color solution | Solution | Pale yellow color solution | Solution | Clear solution | Solution | Clear solution |

Physical criteria (dissolution status, colour) of solution with miglustat 300mg/ml alone or combined with flavours were evaluated. According to the results summarized in Table 12, formulation S with red currant remained a clear solution at all storage conditions. Formulations J with blackcurrant presented a pale yellow or brown color at all tested storage conditions.

**Table 12: Physical description for combination of active and inactive ingredients (flavours)**

| **Formulations** | **Condition/ Physical Description Initial, 60°C, 40°C/75%RH and 25°C/60%RH (closed) of Active + Inactive Ingredient** | | | | | | | | | |
|---|---|---|---|---|---|---|---|---|---|---|
| | **Initial** | **60°C** | **40°C/75%RH** | | | | **25°C/60%RH** | | | |
| | | **1 week** | ***1 week** | **2 weeks** | ***3 weeks** | **4 weeks** | ***1 week** | **2 weeks** | ***3 weeks** | **4 weeks** |
| J-Miglustat + Black currant | Clear Solution | Pale brown color Solution | Solution | Pale brown color solution | Solution | Pale brown color solution | Solution | Pale yellow colored solution | Solution | Pale yellow colored solution |
| S-Miglustat + Red Currant | Clear Solution | Clear Solution | Solution | Clear Solution | Solution | Clear Solution | Solution | Clear Solution | Solution | Clear Solution |

Physical criteria (dissolution status, colour) of solution with miglustat 300mg/ml alone or combined with viscosity modifiers were evaluated. According to the results summarized in Table 13, after few weeks crystal formation appears at all storage conditions. Formulation N with Carbopol becomes a gel at 25°C/60%HR after 4 weeks or a thick gel at 40°C/75%HR after 4 weeks.

**Table 13: Physical description for combination of active and inactive ingredients (flavours)**

| **Formulations** | **Condition/ Physical Description Initial, 60°C, 40°C/75%RH and 25°C/60%RH (closed) of Active + Inactive Ingredient** | | | | | | | | | |
|---|---|---|---|---|---|---|---|---|---|---|
| | **Initial** | **60°C** | **40°C/75%RH** | | | | **25°C/60%RH** | | | |
| | | **1 week** | ***1 week** | **2 weeks** | ***3 weeks** | **4 weeks** | ***1 week** | **2 weeks** | ***3 weeks** | **4 weeks** |
| M-Miglustat + Macrogol 400 (PEG-400) | Viscous solution | Viscous clear solution | Viscous solution | Viscous clear solution | Viscous solution | Viscous clear solution with crystal formation at the bottom of the vial | Solution | Viscous clear solution | Solution | Viscous clear solution with crystal formation at the bottom of the vial |
| N-Miglustat + Carbopol 971P | Viscous solution | Gel formation | Solution | Thick gel formation | Solution | Thick gel formation | Viscous gel like | Gel formation | Viscous gel like | Gel formation |

### Impurities investigation

Total amount of impurities in solutions of miglustat at 300 mg/mL alone or combined with each acidic buffer were measured. According to the results summarized in Table 13, solution B, C and D presented an increase of the amount of impurities after 4 weeks in a closed recipient at 40°C/75%HR and at 25°C/60%HR.

The solution with tartaric acid was stable and no additional impurities appeared after 2 weeks or 4 weeks at 40°C/75%HR or 25°C/60%HR. No interference in the peak.

**Table 13: Quantification of related substances for solution of miglustat alone and combination with acidic buffers**

| **Formulations** | **Total % Related Substances** | | | | | |
|---|---|---|---|---|---|---|
| | **Initial** | **60°C 1 week** | **(40°C/75%RH) 2 weeks** | **(25°C/60%RH) 2 weeks** | **(40°C/75%RH) 4 weeks** | **(25°C/60%RH) 4 weeks** |
| B-Miglustat + Sodium Ascorbate | 0.203 | 0.264 # | 0.177 # | 0.251 | 0.459 | 0.319 |
| C-Miglustat + Ascorbic acid | 0.218 | 0.170 # | 0.207 # | 0.346 | 0.317 | 0.349 |
| D-Miglustat + Sodium Ascorbate + Ascorbic acid | 0.268 | 0.145 # | 0.360 # | 0.447 | 0.350 | 0.409 |
| F-Miglustat + Tartaric Acid | 0.228 | 0.339 | 0.195 | 0.148 | 0.270 | 0.257 |
| P-Miglustat + Hydrochloric acid | 0.249 | 0.307 | 0.265 | 0.390 | 0.127 | 0.212 |

| | | | | | | |
|---|---|---|---|---|---|---|
| # For those 3 solutions, the amount of impurity of degradation was not recorded at 60°C and the 2^{nd} week at 40°C/75%HR because of unknown impurity peaks that interfered with DBU impurity peak (API impurity). | | | | | | |

Total amount of impurities in solutions of miglustat at 300 mg/mL alone or combined with each preservative were measured. According to the results in Table 14, the solution H with sodium metabisulphite presented an increase of impurities quantity at 40°C/75%HR and at 25°C/60%HR in weeks 2 and 4.

For the solution I with sodium benzoate, a small increase of impurities appeared at 40°C/75%HR the 4^{th} week but no significant changes at 25°C/60%HR.

**Table 14: Quantification of related substances for solution of miglustat alone and combination with preservatives**

| **Formulations** | **Total % Related Substances** | | | | | |
|---|---|---|---|---|---|---|
| | **Initial** | **60°C 1 week** | **(40°C/75%RH) 2 weeks** | **(25°C/60%RH) 2 weeks** | **(40°C/75%RH) 4 weeks** | **(25°C/60%RH) 4 weeks** |
| H-Miglustat + Sodium Metabisulphite | 0.228 | 0.352 | 0.456 | 0.329 | 0.357 | 0.319 |
| I-Miglustat + Sodium Benzoate | 0.282 | 0.224 | 0.266 | 0.189 | 0.360 | 0.243 |

Total amount of impurities in solutions of miglustat at 300 mg/mL alone or combined with each sweetener were measured. According to the results in Table 15, in the solution K with aspartame, a high amount of impurity was observed at every time point and every environmental conditions even at the beginning of the study (initial).

For the solution L with the acesulfame potassium, the measurement of impurities quantity was very difficult and no robust analytical method was available to quantify the related substances quantity. No workable results were available for this solution.

**Table 15: Quantification of related substances for solution of miglustat alone and combination with sweeteners**

| **Formulations** | **Total % Related Substances** | | | | | |
|---|---|---|---|---|---|---|
| | **Initial** | **60°C 1 week** | **(40°C/75%RH) 2 weeks** | **(25°C/60%RH) 2 weeks** | **(40°C/75%RH) 4 weeks** | **(25°C/60%RH) 4 weeks** |
| K-Miglustat + Aspartame | 0.801 | 1.191 | 1.543 | 0.909 | 1.109 | 0.794 |
| L-Miglustat + Acesulfame Potassium | - | - | - | - | -- | -- |

Total amount of impurities in solutions of miglustat at 300 mg/mL alone or combined with each flavour were measured. Results are presented in Table 16. For the solution J, with black currant, results were not significant as analytical issues appeared. For the solution S, with red currant, no analytical issues appeared. No increased in impurity at 40°C/75%HR or 25°C/60%HR were observed. A slight increase was observed at 60°C which is expected at the temperature.

**Table 16: Quantification of related substances for solution of miglustat alone and combination with flavours**

| **Formulations** | **Total % Related Substances** | | | | | |
|---|---|---|---|---|---|---|
| | **Initial** | **60°C 1 week** | **(40°C/75%RH) 2 weeks** | **(25°C/60%RH) 2 weeks** | **(40°C/75%RH) 4 weeks** | **(25°C/60%RH) 4 weeks** |
| J-Miglustat + Black currant (Firmenich) | 0.559 | 0.442 | 0.191 | 0.501 | 0.196 | 0.284 |
| S-Miglustat + Red Currant | 0.329 | 0.414 | 0.262 | 0.235 | 0.253 | 0.270 |

Total amount of impurities in solutions of miglustat at 300 mg/mL alone or combined with each flavour were measured. Results are presented in Table 16. For the solution M, with PEG400, results were not significant as analytical issues appeared. For the solution N, with Carbopol 971P, a very significant increase of impurities appears at all storage conditions since the initial time.

**Table 16: Quantification of related substances for solution of miglustat alone and combination with flavours**

| **Formulations** | **Total % Related Substances** | | | | | |
|---|---|---|---|---|---|---|
| | **Initial** | **60°C 1 week** | **(40°C/75%RH) 2 weeks** | **(25°C/60%RH) 2 weeks** | **(40°C/75%RH) 4 weeks** | **(25°C/60%RH) 4 weeks** |
| M-Miglustat + Macrogol 400 (PEG 400) | 0.246 | 0.229 | 0.297 | 0.202 | 0.211 | 0.237 |
| N-Miglustat + Carbopol 971P | 6.405 | 5.974 | 7.695 | 5.695 | 9.888 | 9.592 |

Conclusion: based on the DEC Study, proposed excipients like Tartaric acid, Sodium Benzoate, and Red currant, were found to be compatible with Miglustat in binary mixture for up to 4 weeks in Accelerated conditions 40°C/75%RH, RT condition 25°C/60%RH, and also 60°C 1 week data.

However, PEG 400, Hydrochloric acid, Acesulfame Potassium were found not compatible, for the reasons mentioned above.

### Taste evaluation by experimenters

Each experimenter was allowed to taste the 3 solutions and to assign a grade for each one, allowing to compare the preference in between the different formulations. As presented in Table 17, the best mean rating was attributed to formulation 632, corresponding to the red currant flavour.

**Table 13: Means of the global grade for each formulation tested by the experimenters**

| Formulation code | Mean of global grade (from 0 for the worst and 20 for the best taste) |
|---|---|
| 632 (red currant) | 11,5 |
| 126 (black currant) | 11 |
| 028 (grapefruit) | 9,16 |

### Evaluation of pH and appearance of the formulations

All solutions had a pH under 5 which is mandatory for the efficacy of the sodium benzoate. Only the solution F12 was clear. All solutions with acesulfame potassium were hazy or turbid. Only the solution F12 was clear.

**Table 18: Appearance and pH results for each formulation**

| | *F-12* | *F-13* | *F-20* | *F-21* | *F-26* | *F-27* | *F-28* |
|---|---|---|---|---|---|---|---|
| | *mg*/*ml* | | | | | | |
| *Miglustat* | *250.00* | *250.00* | *250.00* | *250.00* | *250.00* | *250.00* | *250.00* |
| *Tartaric acid* | *150.0* | *150.00* | *100.0* | *100.0* | *88.2* | *88.2* | *88.2* |
| *Sodium benzoate* | *3.00* | *3.00* | *2.30* | *2.30* | *2.30* | *2.30* | *2.30* |
| *Acesulfame K* | *-* | *5.00* | *-* | *-* | *4.0* | *4.0* | *4.0* |
| *Taste sense SR plus LQD* | *-* | *-* | *-* | *1.0* | *-* | *1.0* | *1.0* |
| *Taste sense SR PDR* | *-* | *-* | *1.0* | *-* | *1.0* | *-* | *-* |
| *Magna sweet MM110* | *28.0* | *-* | *29.5* | *29.5* | *-* | *-* | *29.5* |
| *Red currant* | *6.0* | *6.0* | *13.0* | *13.0* | *13.0* | *13.0* | *13.0* |
| *Water* | *q.s* | *q.s* | *q.s* | *q.s* | *q.s* | *q.s* | *q.s* |
| *pH* | *3.47* | *3.50* | *4.26* | *4.22* | *4.79* | *4.81* | *4.84* |
| *Appearance* | *Clear* | *Turbid* | *Hazy* | *Hazy* | *Hazy* | *Hazy* | *Hazy* |

### Distance comparisons between formulation batch trials and miglustat alone

The 7 solutions containing Miglustat were prepared (see Table 4 describing the content of each solution). The E-tongue system measures the distances between the solution of reference (F-18 = miglustat alone at 250 mg/mL) and the tested formulations.

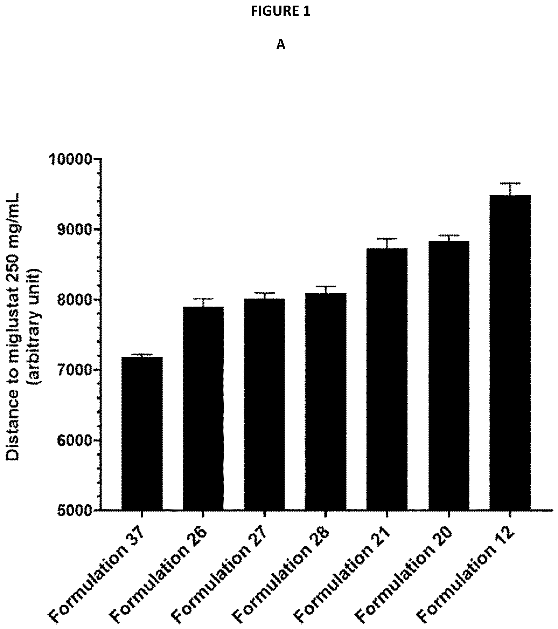

As shown on figure 1A, it was observed that the solution 37 (Miglustat 250mg/mL + tartaric acid + sodium benzoate) was the closest to miglustat alone (F-18) and solution 12 (Miglustat 250mg/mL + tartaric acid + sodium benzoate + Magna sweet + red currant) was the most distant from miglustat alone in term of taste according to e-tongue measurements.

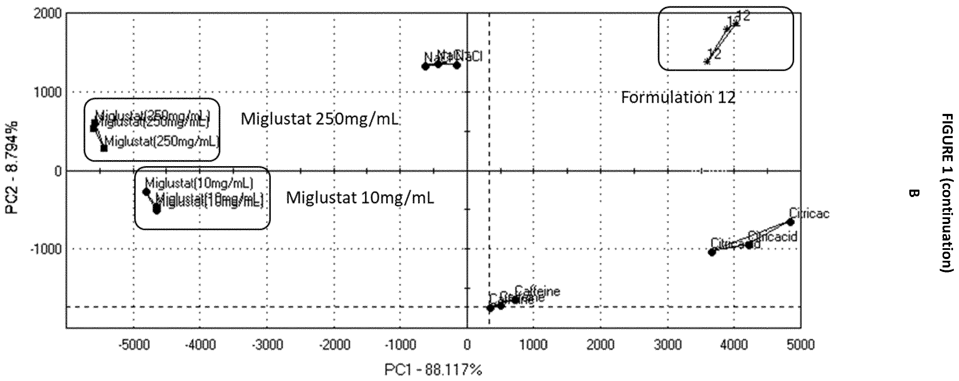

Also, figure 1B shows that the concentration of Miglustat does not modify its aromatic profile. The aromatic profile of formulation 12 is however completely different, even if it contains high amount of miglustat (250 mg/mL).

### Profiling for miglustat alone and the selected formulation

Miglustat alone at 250 mg/mL (formulation F-18) was compared to solutions of sodium chloride, caffeine, sucrose and citric acid at 10 mg/mL each to obtain its aromatic profile.

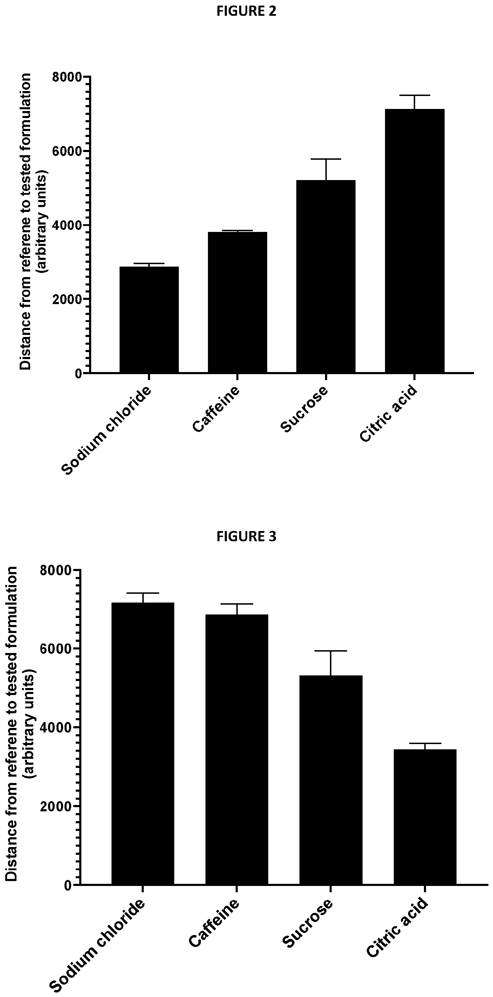

According to the distance analyzed by the e-tongue system presented above, miglustat seems to be closer to sodium chloride and caffeine and farer than sucrose and citric acid (figure 2).

Formulation F-12 was the only clear solution and the farthest form the miglustat alone according to e-tongue results. This solution was compared to solutions of sodium chloride, caffeine, sucrose and citric acid, at 10 mg/mL each, to understand its flavor profile. According to the distance analysed by the e-tongue system presented in Figure 3, the formulation F-12 seems to be more distant to sodium chloride and closer to citric acid which is the opposite of the miglustat alone.

### Example of a solution according to the invention

| **Ingredients** | **mg/mL** | **%W/W** |
|---|---|---|
| **Miglustat** | 250.00 | 57.21 |
| **Tartaric acid** | 150.00 | 34.32 |
| **Sodium Benzoate** | 3.00 | 0.68 |
| **Magna sweet MM 110** | 28.00 | 6.41 |
| **Red Currant** | 6.0 | 1.37 |
| **Water** | q.s | -- |

The microbiological stability of this solution has been tested : the solution remained perfectly sterile when kept for one month at room temperature in a closed recipient.

### BIBLIOGRAPHIC REFERENCES

Amiri M. et Naim H.Y., Miglustat-induced intestinal carbohydrate malabsorption is due to the inhibition of -glucosidases, but not -galactosidases J. Inherit Metab Dis 2012, 35:949-954
Amiri M. et Naim N.Y. Long term differential consequences of miglustat therapy on intestinal disaccharidases J. Inherit Metab Dis 2014, 37:929-937
Belmatoug N et al, Gastrointestinal disturbances and their management in miglustat-treated patients. J. Inherit Metab Dis 2011, 34:991-1001
Getty A.L. and Pearce D.A., 2011 Interactions of the proteins of neuronal ceroid lipofuscinosis: clues to function. Cell Mol Life Sci. 2011 Feb;68(3):453-74
Lyseng-Williamson K.A. et al, Miglustat: A Review of Its Use in Niemann-Pick Disease Type C. Drugs 2014, 74:61-74
Remenova et al, A double-blind, randomized, placebo- controlled trial studying the effects of Saccharomyces boulardii on the gastrointestinal tolerability, safety, and pharmacokinetics of miglustat, Journal of rare diseases, 2015; 10:81
Treem WR et al, Evaluation of liquid Yeast-derived sucrase enzyme replacement in patients with sucrase-isomaltase deficiency, Gastroenterology, 1993; 105:1061-1068

## Claims

1. An acidic liquid formulation for oral administration, said formulation containing at least 150 mg/mL of Miglustat, said liquid formulation containing :
- sodium benzoate as preservative,
- tartaric acid so as to maintain the pH of the formulation comprised between 3 and 5,
- a sweetener, and
- optionally a flavour.

2. The liquid formulation of claim 1, wherein said sweetener is a mix of glycerin and monoammonium glycyrrhizate.

3. The liquid formulation of any one of claims 1 to 2, wherein said flavour is chosen in the group consisting of: ananas, anise, apple, apricot, banana, blackberry, blueberry, caramel, cherry, chocolate, coconut, coffee, cocoa, cola, cranberry, blackcurrant (cassis), redcurrant (or red currant), grape, grapefruit, grenadine, lemon, maple, mint, orange, walnut, passion fruit, peach, pear, pineapple, plum, prune, raspberry, strawberry, tangerine, tutti frutti, vanilla.

4. The liquid formulation of any one of claims 1 to 3, wherein said flavour is an acidic flavour, typically red currant or orange.

5. The liquid formulation of any one claims 1 to 4, containing between 0.5 and 100 mg/mL of sodium benzoate.

6. The liquid formulation of any one claims 1 to 5, containing between 50 and 200 mg/mL of tartaric acid.

7. The liquid formulation of any one claims 1 to 6, containing between 0.1% to 10% by weight of sweetener, preferably of a mix of glycerin and monoammonium glycyrrhizate in a 10/1 ratio.

8. The liquid formulation of any one of claims 1 to 7, containing between 0.01 and 10 % of said flavour.

9. The liquid formulation of any one of claims 1 to 8, containing :
- at least 150 mg/mL of Miglustat,
- between 0.5 and 100 mg/mL of sodium benzoate,
- between 50 and 200 mg/mL of tartaric acid, so that the final pH of the formulation is comprised between 3 and 5,
- between 0.01% to 10% by weight, preferably between 1% and 10% by weight, of a mix of glycerin and monoammonium glycyrrhizate in a 10/1 ratio, and
- between 0.1 and 10 % of red currant or orange flavour.

10. The liquid formulation of any one of claims 1 - 9, wherein it does not contain any carbohydrates, nor any taste enhancer.

11. The liquid formulation of any one of claims 1 - 10, wherein it contains between 250 mg/mL and 300 mg/mL of Miglustat.

12. A liquid formulation as defined in any one of claims 1 - 11, for use as a medicament.

13. A liquid formulation as defined in any one of claims 1 - 11, for use in the treatment of lysosomal storage disorders, preferably in the treatment of Neuronal Ceroid Lipofuscinosis or of the Niemann Pick C disease or of the Gaucher disease.

14. The liquid formulation for use according to claim 13, wherein it is administered to patients suffering from Neuronal Ceroid Lipofuscinosis 3.

15. A combination product comprising the liquid formulation as defined in any one of claims 1-11 and an activator of intestinal disaccharidase enzyme, for simultaneous, separated or staggered use in the treatment of lysosomal storage disorders, preferably in the treatment of children suffering from the Batten Disease, said activator being preferably an heterologous small chemical compound, an enzyme protein, or a living organism that enhances the activity or the expression level of at least one intestinal disaccharidase enzyme chosen among: invertase (EC 3.2.1.26), maltase (EC 3.2.1.20), trehalase (EC 3.2.1.18), sucrase-isomaltase (SI, EC 3.2.1.48 and EC 3.2.1.10 respectively), β-galactosidase (EC 3.2.1.21), lactase- phlorizin hydrolase (LPH, EC 3.2.1.108), sacrosidase, amylase (EC 3.2.1.1) and amyloglucosidase (EC 3.2.1.3).

## Patentansprüche

1. Saure flüssige Formulierung zur oralen Verabreichung, wobei die Formulierung mindestens 150 mg/ml Miglustat enthält, wobei die flüssige Formulierung Folgendes enthält:
- Natriumbenzoat als Konservierungsmittel,
- Weinsäure, um den pH-Wert der Formulierung zwischen 3 und 5 zu halten,
- ein Süßungsmittel, und
- wahlweise ein Aroma.

2. Flüssige Formulierung nach Anspruch 1, wobei das Süßungsmittel ein Gemisch aus Glycerin und Monoammoniumglycyrrhizat ist.

3. Flüssige Formulierung nach einem der Ansprüche 1 bis 2, wobei das Aroma ausgewählt ist aus der Gruppe, die besteht aus: Ananas, Anis, Apfel, Aprikose, Banane, Brombeere, Heidelbeere, Karamell, Kirsche, Schokolade, Kokosnuss, Kaffee, Kakao, Cola, Cranberry, Schwarze Johannisbeere (Cassis), Rote Johannisbeere (oder rote Johannisbeere), Traube, Grapefruit, Grenadine, Zitrone, Ahorn, Minze, Orange, Walnuss, Passionsfrucht, Pfirsich, Birne, Ananas, Pflaume, Zwetschge, Himbeere, Erdbeere, Mandarine, Tutti Frutti, Vanille.

4. Flüssige Formulierung nach einem der Ansprüche 1 bis 3, wobei das Aroma ein saures Aroma, typischerweise rote Johannisbeere oder Orange, ist.

5. Flüssige Formulierung nach einem der Ansprüche 1 bis 4, die zwischen 0,5 und 100 mg/ml Natriumbenzoat enthält.

6. Flüssige Formulierung nach einem der Ansprüche 1 bis 5, die zwischen 50 und 200 mg/ml Weinsäure enthält.

7. Flüssige Formulierung nach einem der Ansprüche 1 bis 6, die zwischen 0,1 Gewichts-% und 10 Gewichts-% Süßungsmittel, vorzugsweise ein Gemisch aus Glycerin und Monoammoniumglycyrrhizat in einem Verhältnis von 10:1, enthält.

8. Flüssige Formulierung nach einem der Ansprüche 1 bis 7, die zwischen 0,01 und 10 % des Aromas enthält.

9. Flüssige Formulierung nach einem der Ansprüche 1 bis 8, die Folgendes enthält:
- mindestens 150 mg/ml Miglustat,
- zwischen 0,5 und 100 mg/ml Natriumbenzoat,
- zwischen 50 und 200 mg/ml Weinsäure, derart dass der endgültige pH-Wert der Formulierung zwischen 3 und 5 beträgt,
- zwischen 0,01 Gewichts-% und 10 Gewichts-%, vorzugsweise zwischen 1 Gewichts-% und 10 Gewichts-%, eines Gemischs aus Glycerin und Monoammoniumglycyrrhizat in einem Verhältnis von 10:1, und
- zwischen 0,1 und 10 % rotes Johannisbeeren-oder Orangen-Aroma.

10. Flüssige Formulierung nach einem der Ansprüche 1 bis 9, wobei sie keine Kohlenhydrate oder Geschmacksverstärker enthält.

11. Flüssige Formulierung nach einem der Ansprüche 1 bis 10, wobei sie zwischen 250 mg/ml und 300 mg/ml Miglustat enthält.

12. Flüssige Formulierung nach einem der Ansprüche 1 bis 11 zur Verwendung als ein Arzneimittel.

13. Flüssige Formulierung nach einem der Ansprüche 1 bis 11 zur Verwendung bei der Behandlung von lysosomalen Speicherkrankheiten, vorzugsweise bei der Behandlung von neuronaler Ceroid-Lipofuszinose oder der Niemann-Pick-Krankheit Typ C oder Morbus Gaucher.

14. Flüssige Formulierung nach Anspruch 13, wobei sie Patienten verabreicht wird, die an neuronaler Ceroid-Lipofuszinose Typ 3 leiden.

15. Kombinationsprodukt, das die flüssige Formulierung nach einem der Ansprüche 1 bis 11 und einen Aktivator von Disaccharidase-Darmenzym umfasst, zur gleichzeitigen, getrennten oder gestaffelten Verwendung bei der Behandlung von lysosomalen Speicherkrankheiten, vorzugsweise bei der Behandlung von Kindern, die an Morbus Batten leiden, wobei der Aktivator vorzugsweise eine heterologe kleine chemische Verbindung, ein Enzymprotein oder ein lebender Organismus ist, die, das bzw. der die Aktivität oder das Expressionsniveau mindestens eines Disaccharidase-Darmenzyms erhöht, das ausgewählt ist aus: Invertase (EC 3.2.1.26), Maltase (EC 3.2.1.20), Trehalase (EC 3.2.1.18), Sucrase-Isomaltase (SI, EC 3.2.1.48 bzw. EC 3.2.1.10), β-Galactosidase (EC 3.2.1.21), Lactase-Phlorizin-Hydrolase (LPH, EC 3.2.1.108), Sacrosidase, Amylase (EC 3.2.1.1) und Amyloglucosidase (EC 3.2.1.3).

## Revendications

1. Une formulation liquide acide pour administration orale, ladite formulation contenant au moins 150 mg/mL de Miglustat, ladite formulation liquide contenant :
- du benzoate de sodium comme conservateur,
- de l'acide tartrique afin de maintenir le pH de la formulation compris entre 3 et 5,
- un édulcorant, et
- optionnellement un arôme.

2. La formulation liquide de la revendication 1, dans laquelle ledit édulcorant est un mélange de glycérine et de glycyrrhizate de monoammonium.

3. La formulation liquide de l'une des revendications 1 à 2, dans laquelle ledit arôme est choisi dans le groupe constitué de : ananas, anis, pomme, abricot, banane, mûre, myrtille, caramel, cerise, chocolat, noix de coco, café, cacao, cola, canneberge, cassis, groseille, raisin, pamplemousse, grenadine, citron, érable, menthe, orange, noix, fruit de la passion, pêche, poire, ananas, prune, pruneau, framboise, fraise, mandarine, tutti frutti, vanille.

4. La formulation liquide de l'une des revendications 1 à 3, dans laquelle ledit arôme est un arôme acide, typiquement de la groseille ou de l'orange.

5. La formulation liquide de l'une des revendications 1 à 4, contenant entre 0,5 et 100 mg/mL de benzoate de sodium.

6. La formulation liquide de l'une des revendications 1 à 5, contenant entre 50 et 200 mg/ml d'acide tartrique.

7. La formulation liquide de l'une des revendications 1 à 6, contenant entre 0,1 % et 10 % en poids d'édulcorant, de préférence un mélange de glycérine et de glycyrrhizate de monoammonium dans un rapport de 10/1.

8. La formulation liquide de l'une des revendications 1 à 7, contenant entre 0,01 et 10 % dudit arôme.

9. La formulation liquide de l'une des revendications 1 à 8, contenant :
- au moins 150 mg/mL de Miglustat,
- entre 0,5 et 100 mg/mL de benzoate de sodium,
- entre 50 et 200 mg/mL d'acide tartrique, de sorte que le pH final de la formulation soit compris entre 3 et 5,
- entre 0,01 % et 10 % en poids, de préférence entre 1 % et 10 % en poids, d'un mélange de glycérine et de glycyrrhizate de monoammonium dans un rapport de 10/1, et
- entre 0,1 et 10 % d'arôme de groseille ou d'orange.

10. La formulation liquide de l'une des revendications 1 à 9, dans laquelle elle ne contient pas de glucides, ni d'exhausteurs de goût.

11. La formulation liquide de l'une des revendications 1 à 10, dans laquelle elle contient entre 250 mg/ml et 300 mg/ml de Miglustat.

12. Une formulation liquide telle que définie dans l'une quelconque des revendications 1 à 11, pour utilisation en tant que médicament.

13. Une formulation liquide telle que définie dans l'une quelconque des revendications 1 à 11, pour utilisation dans le traitement des troubles du stockage lysosomal, de préférence dans le traitement de la lipofuscinose céroïde neuronale ou de la maladie de Niemann Pick C ou de la maladie de Gaucher.

14. La formulation liquide pour utilisation selon la revendication 13, dans laquelle elle est administrée à des patients souffrant de lipofuscinose céroïde neuronale 3.

15. Un produit de combinaison comprenant la formulation liquide telle que définie dans l'une quelconque des revendications 1 à 11 et un activateur de l'enzyme disaccharidase intestinale, pour une utilisation simultanée, séparée ou échelonnée dans le traitement des troubles du stockage lysosomal, de préférence dans le traitement des enfants souffrant de la maladie de Batten, ledit activateur étant de préférence un petit composé chimique hétérologue, une protéine enzymatique, ou un organisme vivant qui augmente l'activité ou le niveau d'expression d'au moins une enzyme disaccharidase intestinale choisie parmi : invertase (EC 3.2.1.26), maltase (EC 3.2.1.20), tréhalase (EC 3.2.1.18), sucrase-isomaltase (SI, EC 3.2.1.48 et EC 3.2.1.10 respectivement), β-galactosidase (EC 3.2.1.21), lactase-phlorizin hydrolase (LPH, EC 3.2.1.108), sacrosidase, amylase (EC 3.2.1.1) et amyloglucosidase (EC 3.2.1.3).
